# EUROPEAN PATENT SPECIFICATION

(11) **EP 2 919 705 B1**
(45) Date of publication and mention of the grant of the patent: **23.06.2021**
(21) Application number: 13799436.4
(22) Date of filing: 14.11.2013
(51) Int. Cl.: A61C 13/00, A61K 6/887

(54) **THREE-DIMENSIONAL FABRICATING MATERIAL SYSTEMS FOR PRODUCING DENTAL PRODUCTS**
DREIDIMENSIONALE KONSTRUKTIONSMATERIALSYSTEME ZUR HERSTELLUNG DENTALER PRODUKTE
SYSTÈMES DE MATÉRIAU DE FABRICATION TRIDIMENSIONNELLE POUR PRODUIRE DES PRODUITS DENTAIRES

(30) Priority: 14.11.2012 US 201261726317 P
(43) Date of publication of application: 23.09.2015
(73) Proprietor: DENTSPLY SIRONA Inc., York, PA 17401-2991 (US)
(72) Inventor: SUN, Benjamin, Jiemin, York, PA 17402 (US); KENNEDY, Christopher, R., New Freedom, PA 17349-9169 (US); SUNDAR, Veeraraghavan, Carmel, IN 46032 (US); LICHKUS, Andrew, M., York, PA 17404 (US)
(74) Representative: Hartz, Nikolai
(86) International application number: PCT/US2013/070099
(87) International publication number: WO 2014/078537

(56) References cited:
- EP-A1- 0 630 640
- EP-A1- 0 630 640
- WO-A1-2006/053154
- WO-A1-2006/053154
- DE-A1- 19 617 876
- DE-A1- 19 617 876
- US-A1- 2002 127 345
- US-A1- 2002 127 345
- US-B1- 6 322 728
- US-B1- 6 322 728
- US-B1- 6 921 500
- US-B1- 6 921 500

## Description

### THE CROSS-REFERENCE TO RELATED APPLICATIONS

This patent application claims the benefit of and priority to U.S. Provisional Patent Application Ser. No. 61/726,317, filed on November 14, 2012.

### TECHNICAL FIELD

The present invention relates generally to rapid prototyping systems for making dental devices such as, for example, artificial teeth, dentures, splints, veneers, inlays, onlays, copings, frame patterns, crowns and bridges, models, appliances and the like. More particularly, using light beam irradiation, such as stereolithography (SLA) or DLP (Digital Light Processor, such as Perfactory system from EnvisionTec) to build-up the dental devices as three-dimensional objects from novel liquid resins of this invention. SLA using laser beam traces out the shape of each layer and hardens the photosensitive resin in a vat. The Perfactory system builds three-dimensional objects by using the Digital Light Processor (DLP) projector to project sequential voxel planes into liquid resin, which then caused the liquid resin to cure.

### BACKGROUND

In general, rapid prototyping refers to a conventional manufacturing process used to make parts, wherein the part is built on a layer-by-layer basis using layers of hardening material. Per this technology, the part to be manufactured is considered a series of discrete cross-sectional regions which, when combined together, make-up a three-dimensional structure. The building-up of a part layer-by-layer is very different than conventional machining technologies, where metal or plastic pieces are cut and drilled to a desired shape. In rapid prototyping technology, the parts are produced directly from computer-aided design (CAD) or other digital images. Software is used to slice the digital image into thin cross-sectional layers. Then, the part is constructed by placing layers of plastic or other hardening material on top of each other. There are many different techniques that can be used to combine the layers of structural material. A curing step may be required to fully cure the layers of material.

Ink-jet printing technology is a rapid prototyping method that can be used to fabricate the three-dimensional object. In one well known ink-jet printing method that was developed at Massachusetts Institute of Technology, as described in Sachs et al., US Patent 5,204,055, printer heads are used to discharge a binder material onto a layer of powder particulate in a powder bed. The powdered layer corresponds to a digitally superposed section of the object that will be produced. The binder causes the powder particles to fuse together in selected areas. This results in a fused cross-sectional segment of the object being formed on the platform. The steps are repeated for each new layer until the desired object is achieved. In a final step, a laser beam scans the object causing the powdered layers to sinter and fuse together. In another ink-jet printing process, as described in Sanders, US Patents 5,506,607 and 5,740,051, a low-melting thermoplastic material is dispensed through one ink-jet printing head to form a three-dimensional object. A second ink-jet printer head dispenses wax material to form supports for the three-dimensional object. After the object has been produced, the wax supports are removed, and the object is finished as needed.

Leyden et al., US Patents 6,660,209 and 6,270,335 disclose an ink-jet printing method using commercial print heads having multiple orifices (jets) to selectively fire droplets of hot melt, radiation-curable material onto a substrate. Each orifice can be equipped with a piezoelectric element that causes a pressure wave to propagate through the material when electric current is applied. The print head moves along a scan path selectively depositing the flowable material onto the substrate. In a subsequent step, light radiation is used to cure the material.

Yamane et al., US Patent 5,059,266 discloses an ink-jetting method, whereby a photosetting or thermosetting resin is jetted along a flight passage of the material to a stage to thereby laminate the material on the stage, changing at least one of a jetting direction of the material along the flight passage and a jetting amount of the material, thereby controlling a jetting operation of the material, and exposing the laminated material to light to cure the material, thereby forming the article,

Bredt et al., US Patent 5,902,441 describes another ink-jet printing method, which involves applying a layer of powder particles containing an activatable adhesive onto a flat surface that can be indexed downward. The ink-jet printer introduces an activating fluid onto to the layer of particles in a predetermined pattern. The fluid activates the adhesive in the mixture, causing the particles to adhere together in an essentially solid layer. After the first cross-sectional portion of the article is formed, the movable surface can be indexed downward. Successive layers of the mixture of particles are applied in the same manner to form the desired article.

Oriakhi et al., US Patent Application Publication No. US 2005/0082710 discloses an ink-jet printing method, wherein a particulate blend of reactive glass ionomer particulates, crosslinkable polyacid particulates including polyvinyl pyrrolidone-co-polyacrylic acid, and nanocomposites is spread in a fabrication bin. An ink-jet printer applies an aqueous phase binder onto a predetermined area of the particulate blend to form hydrated cement. A glass-ionomer chemical reaction causes the hydrated cement to harden.

Kapserchik et al., US Patent Application Publication No. US 2004/0094058 discloses an ink-jet printing system using acid-base cements. Layers of powder particulate are deposited on a flat surface. The powders include a base such as a metal oxide or an aluminosilicate glass, a polymeric acid or other acid. The ink-jet printer dispenses an aqueous binder. The basic powder interacts with the acid in the presence of water, causing the formation of an ionically cross-linked hydrogel salt. Formation of the cross-linked hydrogel causes setting of the mixture.

More particularly, ink-jet printing methods for making three-dimensional dental products have been developed and are described in the patent literature.

For example, Moszner et al., US Patent 6,939,489 discloses a process for fabricating three-dimensional dental form pieces for dental restoration and replacement parts using three-dimensional plotting technology. The object is produced in a layered manner by the cutting away of micro drops or micro cords discharged from nozzles in the three-dimensional plotter. The discharged material can be hardened by a variety of mechanisms depending upon the type of material used. This includes cooling of melted material, polycondensation, polyaddition, or thermal-curing, and light radiation. In the '489 Patent, the three-dimensional plotting technology is described as being different than conventional rapid prototyping (selective laser sintering, 3D printing, and stereolithography).

Rheinberger et al., US Patent 7,189,344 discloses a process for producing three-dimensional dental restorative parts, such as full or partial dental prosthesis, using ink-jet printers that are used in the ink-jet printing methods developed by MIT as described above. The process involves spraying a polymerizable material onto a base support in a layer-by-layer manner. Each layer of material is polymerized by a light source prior to the application of the next layer. The polymerizable material is described as being wax-like having up to 70% by weight of at least one of a polymerizable monomer and oligomer; from 0.01 to 10% by weight of a polymerization initiator; and at least 20% by weight of a mixture having a selected one of a wax-like and flowable monomer and a color pigment.

Feenstra, US Patents 6, 921,500 and 6,955,776 disclose an ink-jet printing process for making dental elements such as crowns using a liquid binder and powder bed. The element is produced by applying successive layers of powder and discharging the liquid binder onto the layers using an ink-jet printer. The binder preferably includes nanomeric, inorganic solid particles having polymerizable and/or polycondensable organic groups at their surface. After the binder has been applied to the last layer of powder, any excess, unbound powder is removed. Then, the powdered layers are sintered by heating to a temperature in the range of about 400 to 800°C. The sintering step is performed so that only necks between the powder particles are formed. The resulting sintered dental element is infiltrated by a second phase material, such as glass-ceramic or polymer, which melts at a lower temperature than the material of the dental element. This reduces the porosity of the dental element.

Bordkin et al., US Patent 6,322,728 discloses an ink-jet printing process for making dental restorations by printing a binder into layers of powder. The process involves depositing a layer of ceramic or composite powder material onto a powder bed. The design of the restoration is based on a CAD representation. A binding material is applied onto the ceramic or composite layer. This application of powder/binder material is repeated several times to produce the desired shape of the restoration. After the layering process is completed, the structure is cured to further promote binding of the particles.

The present invention provides novel liquid resin systems for fabricating three-dimensional dental devices using the Digital Light Processor (DLP) projectors or other light beam irradiations, such as stereolithography. Although the DLP method or stereolithography and materials are described primarily herein as being used to make a denture base and teeth, it should be understood that this is for illustration purposes only. The DLP method or stereolithography and materials can be used to make any dental device such as, for example, artificial teeth, dentures, splints, veneers, inlays, onlays, copings, orthodontics, aligners, frame patterns, crowns and bridges and the like. We have provided a general description of this method and material systems as follows. A more detailed description of the methods and materials used to make the dental devices is set forth below.

In this method, a polymerizable liquid resin material or heated resin material as a liquid is loaded into a resin bath of a 3D printer based on a DLP method or stereolithography. In the case of using DLP method, it builds 3D objects by projecting sequential voxel planes into liquid resin (or heated resin), which then polymerizes it to solid. Successive layers of polymerized material are added in this manner until the device is completely fabricated. Then the device, for example, a denture, is washed, finished and fully final cured as needed. The fully cured and polished denture is now ready to be used by the patient.

### SUMMARY OF THE INVENTION

The present invention is defined in the appended claims. The materials of this invention are suitable for dental application and cured to high mechanical strength and have excellent physical properties. Further, these materials have good biocompatibility making it ideal for dental applications. The method for making a three-dimensional dental prosthesis uses a printable composition comprising:
a mixture of
   at least 1% of methyl methacrylate and/or ethyl methacrylate;
5 to 10% a silicone-acrylic-based rubber impact modifier wherein the core is silicone and the shell is PMMA acrylic, which does not dissolve but swells and forms a colloid at room temperature or elevated temperature;
1 to 90% of at least one mono or multifunctional (meth)acrylate;
0 to 60% of at least one inorganic filler;
0 to 60% of at least one organic fillers;
0 to 10% pigments, and
0.01 to 10% of light initiators,
said method either
   (A) comprising the steps of
      a. loading the polymerizable liquid resin material or heated resin material as a liquid into a resin bath of a 3D printer;
      b. applying sequential voxel planes into the liquid resin or heated resin to form a first layer of material, which polymerizes into a solid;
      c. applying one or more successive layers of the polymerized material until a predetermined is formed,
      or
   (B) comprising the steps of
      a. loading the polymerizable liquid resin material or heated resin material as a liquid into a resin bath of a 3D printer based on stereolithography or other light irradiations;
      b. using laser beam or light irradiation tracing out the shape of each layer of the liquid resin or heated resin to form a polymerized solid;
      c. applying one or more successive layers of the polymerized material until a predetermined is formed.

### Printable Polymerizable Materials

A printable polymerizable material is used to make the dental products in accordance with the methods of this invention. By the term, "printable" as used herein, it is meant a material which is flowable (fluid) at a temperature below ambient temperature, at ambient temperature and above ambient temperature.

Flowable material having a flowable temperature in the range of -30°C to 140°C. The following components can be used to prepare the printable polymerizable material in accordance with this invention.

### Polymerizable Acrylic Compounds

Polymerizable acrylic compounds that are used in the compositions of this invention, include methyl methacrylate and/or ethyl methacrylate, as well as at least one mono or multifunctional (meth)acrylate.

The polymerizable acrylic compound may be present in an amount of at least about 10% by weight and preferably at least about 35% by wt the overall polymerizable composition. Furthermore, the polymerizable acrylic compound may be present in an amount of less than about 99.9% by weight, and preferably less than about 95% by wt the overall polymerizable composition. For example, the polymerizable acrylic compound may range from about 10% to about 99.9% by weight, and preferably from about 35 to about 95% by wt the overall polymerizable composition.

### Polymerization System

Printable polymerizable dental materials and compositions of this invention may include one or more initiating systems to cause them to harden promptly. Light polymerizable dental compositions or composites preferably include a light sensitizer, for example camphorquinone, 2,4,6- trimethylbenzoyldiphenylphosphine oxide, or methyl benzoin which causes polymerization to be initiated upon exposure to activating wavelengths of light; and/or a reducing compound, for example tertiary amine.

In one embodiment, a photoactive agent such as, for example, benzophenone, benzoin and their derivatives, or alpha-diketones and their derivatives is added to the composition in order to make it light-curable. A preferred photopolymerization initiator is camphorquinone (CQ). Cationic polymerization initiator, 4-octyloxy-phenyl-phenyl iodonium hexafluoroantimonate (OPPI), can also be used, which initiates ring opening polymerization as well as volume expansion from phase change to reduce the polymerization shrinkage. Photopolymerization can be initiated by irradiating the composition with blue, visible light preferably having a wavelength in the range of about 400 to about 500 nm. A standard dental blue light-curing unit can be used to irradiate the composition. The camphorquinone (CQ) compounds have a light absorbency maximum of between about 400 to about 500 nm and generate free radicals for polymerization when irradiated with light having a wavelength in this range. Photoinitiators selected from the class of acylphosphine oxides can also be used. These compounds include, for example, monoacyl phosphine oxide derivatives, bisacyl phosphine oxide derivatives, and triacyl phosphine oxide derivatives. For example, 2,4,6-trimethylbenzoyl-diphenyl-phosphine oxide (TPO) can be used as the photopolymerization initiator.

In addition to the photoactive agents, the material of this invention may include a polymerization inhibitor such as, for example, butylated hydroxytoluene (BHT); hydroquinone; hydroquinone monomethyl ether; benzoquinone; chloranil; phenol; butyl hydroxyanaline (BHA); tertiary butyl hydroquinone (TBHQ); tocopherol (Vitamin E); and the like. Preferably, butylated hydroxytoluene (BHT) is used as the polymerization inhibitor. The polymerization inhibitors act as scavengers to trap free radicals in the composition and to extend the material's shelf life.

In one embodiment, a material referred to as "ALF" comprising camphorquinone (CQ); butylated hydroxytoluene (BHT); N, N-dimethylaminoneopentyl acrylate, gamma-methacryloxypropyl trimethoxy silane and methacrylic acid can be used in the composition.

The initiating component may be present in an amount of at least 0.05% by weight, and preferably at least about 0.3% by wt the overall polymerizable composition. The overall polymerizable composition may include less than about 20% and more preferably less than about 5% by wt of the initiating component. For example, the initiating component may be present in a range of about 0.05% to about 10%, and preferably from about 0.3% to about 5% by wt of the overall polymerizable composition.

### Fillers

Conventional filler materials such as inorganic fillers, which can be naturally-occurring or synthetic, can be added to the printable polymerizable dental material and composition. Such materials include, but are not limited to, silica, titanium dioxide, iron oxides, silicon nitrides, glasses such as calcium, lead, lithium, cerium, tin, zirconium, strontium, barium, and aluminum-based glasses, borosilicate glasses, strontium borosilicate, barium silicate, lithium silicate, lithium alumina silicate, kaolin, quartz, and talc. Preferably, the silica is in the form of silanized fumed silica. Preferred glass fillers are silanized barium boron aluminosilicate and silanized fluoride barium boron aluminosilicate. Preferably, these inorganic fillers can be suspended in printable polymerizable resin. Organic particles such as poly(methyl methacrylate) (PMMA), highly crosslinked PMMA beads, poly(methyl/ethyl methacrylate), poly(methyl/butyl methacrylate), rubber modified PMMAs, rubber impact modifiers, crosslinked polyacrylates, thermoplastic and crosslinked polyurethanes, grounded polymerized compounds of this invention, polyethylene, polypropylene, polycarbonates and polyepoxides , and the like also can be used as fillers. These organic fillers can be added into printable polymerizable resin described above. Preferably, these organic fillers can dissolve or suspend in printable polymerizable resin.

The inorganic filler particles can be surface-treated with a silane compound or other coupling agent to improve bonding between the particles and resin matrix. Suitable silane compounds include, but are not limited to, gamma-methacryloxypropyltrimethoxysilane, gamma-mercaptopropyltriethoxysilane, gamma-aminopropyltrimethoxysilane, and combinations thereof.

The filler is optional. The filler component may be present in an amount of at least 0% by weight, and more preferably at least about 2% by wt the overall polymerizable composition. Furthermore, the filler component may be present in an amount less than about 75% by weight and more preferably less than about 65% by wt of the overall polymerizable composition. For example, the filler component may be present in a range of about 0 to about 75, and preferrably from about 2 to about 65% by wt of the overall polymerizable composition.

### Pigments

Examples of the inorganic pigment include, but not limited to, black iron oxide, yellow iron oxide, ultramarine blue, brown iron oxide, titanium oxide, zinc flower, zinc oxide, iron oxide, aluminum oxide, silicone dioxide, talc, barium sulfate, calcium sulfate, red oxide, cobalt chrome green, Armenian blue, carbon black, mica, cobalt violet, molybdenum red, titanium cobalt green, molybdate orange, etc. Examples of the organic pigments include Cromophtal Red-BRN 2-napthalenecarboxamide, azo pigments, polyazo pigments, azomethine pigments, isoindoline pigments, anthraquinone pigments, phthalocyanine pigments, benzimidazolone pigments, etc.

Printable polymerizable resins based pigmented materials of this invention contains one or more pigments as coloring or shading agents. The pigments include inorganic pigments and organic pigments. The pigments may be modified to increase the dispersibility. For example, modified pigments having a silane group, a polymerizable silane group, dialkylaminomethyl group or dialkylaminoethylsulfonic acid group are preferred used. In an additional example, inorganic pigments can be surface-treated with a silane compound or other coupling agent to improve bonding between the particles and resin matrix and dispersion in materials. Suitable silane compounds include, but are not limited to, gamma-methacryloxypropyltrimethoxysilane, gamma-mercaptopropyltriethoxysilane, gamma-aminopropyltrimethoxysilane, and combinations thereof.

The term "pigment" refers to visible materials which are not soluble, but are suspended or dispersed as fine particles in the subject materials. The preferred solid pigments are those pigments with fine particles, such as Black Iron Oxide 7053, Yellow Iron Oxide 7055, Titanium Dioxide, Cromophtal Red-BRN 2-napthalenecarboxamide, N,N'-(2-chloro-14-phenylene) bis{4-{(2,5-dichlorophenyl) azo}-3-hydroxy-}, ultramarine blue and brown iron oxide 420. In addition, a fluorescing agent may be included, such as Lumilux Blue LZ fluorescing agent (dihydroxy terepthalate acid ester). The surface of pigments may be organically modified to improve its compatibility to resin matrix. Pigments may also be prepolymerized in resin matrix as small beads or bulk and then grounded to powder, so as to enhance their suspension in low viscose liquid resins. The printable polymerizable materials are applied directly to form dental devices and solidify immediately upon light irradiation/projection, migration of the material is prevented, and dimensional precision is achieved.

Pigmented materials are desirable because they have superior shade stability and stand up to UV light irradiation. This invention overcame the potential pigment separation from dental resins by dispersing the particles in the solution better to prevent settling and by milling the particles to smaller. This invention further overcame the potential pigment separation from dental resins by using nano-dispersed and fine inorganic and organic pigments.

The pigment is optional. Clear formulations do not need any pigments. The pigment component may be present in an amount of at least 0% by weight, and more preferably at least about 0.001% by wt the overall polymerizable composition. The overall polymerizable composition also may include less than about 5% by weight and more preferably less than about 1% by wt of the pigment component. For example, the pigment component may be present in a range of about 0 to about 5%, and preferably from about 0.001 to about 1% by wt of the overall polymerizable composition.

Printable polymerizable dental materials compositions of the invention may include various inorganic and organic fillers, pigments, initiators, catalysts, stabilizers, plasticizers, fibers or their combinations. Preferred stabilizers are butylated hydroxytoluene (BHT) and the methyl ether of hydroquinone (MEHQ). It may also include compounds to introduce radiopaque in the material.

Printable polymerizable dental materials of the invention are able to rapidly solidify upon light irradiation. Rapid solidification provides a combination of flowability and dimensional stability, depending on its temperature prior to polymerization.

### Rubber impact modifier

This invention provides a novel rubber impact modified approach through the use of special selected rubber impact modifier (e.g., S2006, a silicone-acrylic-based rubber from Mitsubishi Rayon Co.) into resin/liquid system, where core-shell (core is silicone, shell is PMMA acrylic) rubber impact modifier does not dissolve but swells and forms a colloid at room temperature or elevated temperature. Surprisingly, this novel approach provides the significantly improved impact resistance and fracture toughness of the resin/liquid system. In accordance with a preferred form of the present invention, novel rubber impact modified dental resin/liquid compositions are provided, which polymerized by known techniques, such as light irradiations, into prosthetic devices possessing chemical and physical properties which are significantly improved over those of the dental devices made from not rubber impact modified resin/liquid composition or even conventional prior art acrylic materials. Notably, dental devices, such as, for example, various prosthetic devices, such as denture bases, produced from novel rubber impact modified dental resin/liquid composition prepared in accordance with the invention are characterized by improved fracture toughness.

Furthermore, denture devices, such as denture bases, produced from novel rubber impact modified resin/liquid compositions of the invention have excellent stain, chemical and solvent resistances. They also have excellent bonding strength to acrylic plastic teeth or other dental devices in the market. In comparison with light curable denture bases, even conventional acrylic denture bases, the denture bases produced in accordance with this invention are characterized by outstanding fracture toughness.

The novel rubber impact modified resin/liquid compositions are formed in accordance with the invention by combining at least a monomer, crosslinking agents for said monomer, at least a rubber impact modifier, which disperses evenly and maintains a homogeneous appearance in this resin/liquid.

Generally, it is preferable for the overall polymerizable composition to include at least one impact modifier. As used herein, like with any other ingredients of the present invention, the term "impact modifier" can include one impact modifier or plural impact modifiers. Various impact modifiers may be employed in the practice of the present invention and often include one or more elastomers. It is generally preferable for the impact modifier to be at least 0.5%, more typically at least 1%, even more typically at least 2%, still more typically at least 3% and even still more typically at least 5% by weight of the overall polymerizable composition and also preferable for the impact modifier to be less than 40%, more typically less than 25% an even more typically less than 15% by weight of the overall polymerizable composition, although higher or lower amounts may be used in particular embodiments. For example, the impact modifier may be present in an amount ranging from about 2% to about 40%, typically from about 3% to about 25%, and preferably from about 5% to about 15% by wt of the overall polymerizable composition.

The rubber impact modifiers are in the form of small particles having average diameters ranging from about 0.01 micron to about 100 microns. Preferably, particles have diameters ranging from 0.02 micron to about 20 microns. More preferably, particles have diameters ranging from 0.05 micron to about 10 micron. The rubber impact modifiers particles are fully dispersed into the monomer, crosslinking agents and the rest of liquid/melted resin. Its hard shells are fully swollen and penetrated by the used monomer/oligomer while the soft cores remain relative intact so as to maintain distinct hard and soft phases and provide adequate suspension in the rest of components in composition and become a part of crosslinked and interpenetrating polymer network. It has been discovered that the composition of this rubber impact modifier and relative proportion of this modifier dramatically affect the impact resistance and fracture toughness of final cured composition as well as the handling properties at uncured stage. This invention provides components for a desired composition to the attainment of the desired properties in the final hardened or cured product produced therefrom, notably the impact resistance and fracture toughness.

The present disclosure may provide for rubber impact modified compositions, which are particularly useful in the production of light curable dental materials, e.g., denture bases, with properties, especially fracture toughness, superior to those of light curable denture bases or even conventional acrylic systems now used in the art. Advantageously, the impact modified compositions of the present invention provide for the introduction of unique homogeneous rubber impact modified liquids/resins, rubber impact modifier, which enhanced the impact strength and fracture toughness of cured product surprisingly.

As used herein, the term core/shell impact modifier may denote an impact modifier wherein a substantial portion (e.g., greater than 30%, 50%, 70% or more by weight) thereof is comprised of a first polymeric material (i.e., the first or core material) that is substantially entirely encapsulated by a second polymeric material (i.e., the second or shell material). The first and second polymeric materials, as used herein, can be comprised of one, two, three or more polymers that are combined and/or reacted together (e.g., sequentially polymerized) or may be part of separate or same core/shell systems.

The first and second polymeric materials of the core/shell impact modifier can include elastomers, polymers, thermoplastics, copolymers, other components, combinations thereof or the like. In preferred embodiments, the first polymeric material, the second polymeric material or both of the core/shell impact modifier include or are substantially entirely composed of (e.g., at least 70%, 80%, 90% or more by weight) one or more thermoplastics. Exemplary thermoplastics include, without limitation, polycarbonate, polyester, polyolefin, polystyrene polypropylene, polyethylene terephthalate, polyvinyl chloride, polyamide, polyethylene, polybutylene terephthalate, acrylonitrile-butadiene-styrene resin, polymethyl methacrylate, or the like, and/or any combination thereof. Desirably, silicone-acrylic-based rubber and/or butadiene-based rubber (e.g., MMA-butadiene-styrene or Acrylonitrile-butadiene-styrene) core/shell impact modifiers may be included to achieve both superior high impact strength and/or excellent weatherability.

Examples of useful core-shell graft copolymers are those where hard containing compounds, such as styrene, acrylonitrile or methyl methacrylate, are grafted onto core made from polymers of soft or elastomeric containing compounds such as butadiene or butyl acrylate. The core polymer, may also include other copolymerizable containing compounds, such as styrene, vinyl acetate, methyl methacrylate, butadiene, isoprene, or the like. The core polymer material may also include a cross linking monomer having two or more nonconjugated double bonds of approximately equal reactivity such as ethylene glycol diacrylate, butylene glycol dimethacrylate, and the like. The core polymer material may also include a graft linking monomer having two or more nonconjugated double bonds of unequal reactivity.

A characteristic of the rubber impact modified liquid/resin is that the rubber impact modifier will be insoluble in, but will absorb or imbibe, the liquid or melted polymerizable monomer component used in the preparation of the rubber impact modified liquid/resin and form a colloid at room temperature or elevated temperature, a homogeneous mixture at room temperature or elevated temperature. A desirable rubber impact modifier may include a multilayered polymer that is constituted of the core layer(s) that contains a composite rubber containing an acrylic component and a silicone component and the shell layer(s). Preferably the multilayered polymer does not contain as the constituent components unreacted epoxy groups and/or unreacted allyl groups, though not required. Rubber impact modifiers can be used in the composition of this disclosure, include, but are not limited to, Metablen S2006, S2001, S2030, SRK200, C223 (all sold from Mitsubishi Rayon Co.), and D440 (sold by Arkema), etc.

### Methods

### 3D Printing using DLP system and 3D printing using stereolithography

In general, these two approaches (DLP printer or Stereolithography printer) can be used for fabricating the three-dimensional object using the materials of this disclosure.

Following each of these approaches, the printable polymerizable material is flowable or heated to form a flowable liquid. The printer builds successive layers of the polymerizable material by projecting or irradiating light onto the building plane and cures to form the denture or other dental device. The resulting denture or other dental device should exhibit excellent mechanical and physical properties, shade and color properties.

Several printable polymerizable materials with different shades and color can be prepared and placed into separate baths. In a case of build a denture, denture base is build from denture base shaded bath layer by layer. This denture base is washed and transferred into a dentin bath to build tooth dentin part of denture teeth on denture base layer by layer. After it is washed and transferred into an enamel bath, where an enamel layer is build layer by layer and forms a final denture device with integral teeth on denture base.

In a case of mass production of denture teeth, multiple teeth can be built by first forming multiple neck parts of denture teeth in neck resin bath, and adding body parts of denture teeth in body resin bath, finally building enamel parts of denture teeth in enamel resin bath and final cure to form multiple denture teeth. Multiple baths at ambient atmosphere and elevated temperature may be used as desired to achieve the desirable esthetics of formed dental devices.

Preferably, high-strength dental products are produced by the methods of this invention. In a preferred embodiment, the printable polymerizable material (with no reinforcing fillers) can be cured from printer to produce the high-strength dental product. By the term, "high-strength" as used herein, it is meant that the products have a flexural modulus of at least 200,000 psi and a flexural strength of at least 5,000 psi. More preferably, the product has a flexural modulus of at least 300,000 psi and a flexural strength of at least 8,000 psi. Most preferably, the product has a flexural modulus of at least 350,000 psi and a flexural strength of at least 12,000 psi. "Flexural strength and flexural modulus" as used herein refers to properties measured according to the methods of ASTM D790 (1997).

Also, as described in the following examples, various formulations of the printable polymerizable materials can be prepared for use in a printing device. It is important that the formulations have sufficiently low viscosity so that they can be handled and cured device can be removed easily from the liquid resin bath (reservoir). At the same time, the formulations must be capable of producing dental products having sufficient mechanical strength and integrity. Several flowable, printable polymerizable materials were prepared with various shades for different applications. The flowable, printable polymerizable materials were successfully, locally cured to form various 3D objects. Several selected examples are shown in the Example Section. The materials of this disclosure were cured in this manner layer by layer and formed 3D dental objects that can be separated from the rest of liquid resin in the bath of 3D printer. Additionally, wash solvents (e.g., ethyl acetate, alcohols, acetone, THF, heptane, etc. or their combinations) may be used to remove uncured resin from 3D dental objects and final cure or heat treatment may be used to enhance their mechanical and physical properties as well as their performance. Air barrier coating or sealer may be used prior to final cure. Inert atmosphere may be used for final cure dental devices or mass production of dental devices (e.g., denture teeth, denture bases, crowns) in a manufacturing environment.

Alternatively, the materials of this disclosure can be made by other means to build 3D objects. In addition, the resin systems developed in this invention can be used in other industries, such as aerospace, animation and entertainment, architecture and art, automotive, consumer goods and packaging, education, electronics, hearing aids, sporting goods, jewelry, medical, manufacturing, etc.

### EXAMPLES

### EXAMPLE 1

### Preparation of Oligomer

A reactor was charged with 1176 grams of trimethyl-1,6-diisocyanatohexane (5.59 mol) and 1064 grams of bisphenol A propoxylate (3.09 mol) under dry nitrogen flow and heated to about 65°C under positive nitrogen pressure. To this reaction mixture, 10 drops of catalyst dibutyltin dilaurate were added. The temperature of the reaction mixture was maintained between 65 °C and 140°C for about 70 minutes and followed by additional 10 drops of catalyst dibutyltin dilaurate. A viscous paste-like isocyanate end-capped intermediate product was formed and stirred for 100 minutes. To this intermediate product, 662 grams (5.09 mol) of 2-hydroxyethyl methacrylate and 7.0 grams of BHT as an inhibitor were added over a period of 70 minutes while the reaction temperature was maintained between 68°C and 90° C. After about five hours stirring under 70 °C, the heat was turned off, and oligomer was collected from the reactor as semi-translucent flexible solid and stored in a dry atmosphere.

### EXAMPLE 2

### Preparation of Monomer

A reaction flask was charged with 700 grams of 1,6-diisocyanatohexane and heated to about 70 °C. under positive nitrogen pressure. To this reactor were added 1027 grams of 2-hydroxyethyl methacrylate, 0.75 gram of catalyst dibutyltin dilaurate and 4.5 grams of butylated hydroxy toluene (BHT). The addition was slow and under dry nitrogen flow over a period of two hours. The temperature of the reaction mixture was maintained between 70°C and 90°C for another two hours and followed by the addition of 8.5 grams of purified water. One hour later, the reaction product was discharged as clear liquid into plastic containers and cooled to form a white solid and stored in a dry atmosphere.

### EXAMPLE 3

### Preparation of Monomer

A reaction flask was charged with 168 grams of 1,6-diisocyanatohexane and heated to about 70°C under a positive nitrogen pressure. To this reactor were added 228 grams of 2-hydroxyethyl acrylate, 0.12 gram of catalyst dibutyltin dilaurate and 0.86 grams of butylated hydroxy toluene (BHT). The addition was slow and under dry nitrogen flow over a period of two hours. The temperature of the reaction mixture was maintained between 70°C and 85°C for another three hours and followed by the addition of 0.9 grams of purified water. One hour later, the reaction product was discharged as clear liquid into plastic containers and cooled to form a white solid and stored in a dry atmosphere.

### EXAMPLE 4

### Preparation of Monomer

A reaction flask was charged with 200 grams of octadecyl isocyanate and heated to about 78°C under a positive nitrogen pressure. To this reactor were added 90.6 grams of 2-hydroxyethyl methacrylate, 0.14 gram of catalyst dibutyltin dilaurate and 0.58 grams of butylated hydroxy toluene (BHT). The addition was slow and under dry nitrogen flow over a period of two hours. The temperature of the reaction mixture was maintained between 70°C and 85°C for another 3 hours, and the reaction product was discharged as clear liquid into plastic containers and cooled to form a white solid and stored in a dry atmosphere.

### EXAMPLE 5

### Preparation of Urethane Monomer (UCDPMAA)

A 500 mL flask was charged with 38.8 grams (0.200 mol) of 1,3-bis(isocyanatomethyl)cyclohexane under dry nitrogen flow and heated to about 60°C under positive nitrogen pressure. To this reaction mixture, 3 drops of catalyst dibutyltin dilaurate were added. A mixture of 22.7 grams of 2-hydroxy-3-phenoxy propyl acrylate, 26.6 grams (0.204 mol) of 2-hydroxyethyl methacrylate, 11.5 grams (0.099 mol) of 2-hydroxyethyl acrylate and 0.10 grams of BHT as an inhibitor were added over a period of 70 minutes while the reaction temperature was maintained between 56°C and 78°C. After about four hours stirring, the heat was turned off, and monomer was collected from the flask as viscous liquid and stored in a dry atmosphere.

### EXAMPLE 6

### Organic filler material

A polymerizable dental material was prepared by stirring at 85°C a liquid mixture of 38.65 grams of oligomer (e.g., about 25 to about 55%, preferably from about 30 to about 45% by wt the organic filler material) made following the procedure of Example 1; 46.5 grams of the compound of Example 2 (e.g., about 30 to about 60, preferably from about 35 to about 55% by wt the organic filler material); 6.5 grams of the compound of Example 3 (e.g., about 0.5 to about 15%, preferably from about 1 to about 10% by wt the organic filler material); 8.0 grams of the compound of Example 4 (e.g., about 0.5 to about 20%, preferably from about 1 to about 15% by wt the organic filler material); and 0.35 grams of 2,4,6- trimethylbenzoyldiphenylphosphine oxide, (Lucirin TPO made by BASF) (e.g., about 0.005 to about 10%, preferably from about 0.05 to about 5% by wt the organic filler material). This material was light cured and subsequently ground to form particulate powder containing particles having an average particle size in the range of about 1 to about 150 micrometers, preferably about 2 to about 50 micrometers. Alternatively, these polymer beads can be made by suspension or emulsion polymerizations.

### EXAMPLE 7

### Composite filler material

A polymerizable dental composite material was prepared by stirring at 85°C a liquid mixture of 4.12 grams of oligomer made following the procedure of Example 1 (e.g., about 0.5 to about 15, preferably from about 1 to about 10% by wt the composite filler material); 4.20 grams of the compound of Example 2 (e.g., about 0.5 to about 15, preferably from about 1 to about 10% by wt the composite filler material); 1.45 grams of the compound of Example 3 (e.g., about 0.05 to about 10, preferably from about 0.5 to about 5% by wt the composite filler material); 5.45 grams of 7,7,9-trimethyl-4,13-dioxo-3,14 dioxa-5,12-diazahexadecane-1,16-diol dimethacrylate (e.g., about 0.5 to about 15, preferably from about 1 to about 10% by wt the composite filler material); 6.00 grams of Ethoxylated bisphenol A dimethacrylate (SR348 from Sartomer Company, Inc.) (e.g., about 0.5 to about 20, preferably from about 1 to about 15% by wt the composite filler material); 2.00 grams of silanated fumed silica (SiO₂) (e.g., about 0.05 to about 15, preferably from about 0.5 to about 10% by wt the composite filler material) having an average particle size of from about 0.01 to about 0.04 micrometers; 62 grams of silanated barium aluminoflurosilicate glass particles BAFG (e.g., about 40 to about 80, preferably from about 50 to about 70% by wt the composite filler material) having an average particle size of from about 0.1 to about 1 micrometer; 14 grams of silanated barium aluminoflurosilicate glass particles BAFG (e.g., about 1 to about 30, preferably from about 5 to about 25% by wt the composite filler material) having an average particle size of from about 1 to about 10 micrometers; and 0.28 grams of visible light initiating solution (e.g., about 0.005 to about 10, preferably from about 0.05 to about 5% by wt the composite filler material) containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane. This material was light cured and subsequently ground to form particulate powder containing particles having an average particle size in the range of about 1 to about 150 micrometers, preferably about 2 to about 50 micrometers. Alternatively, these composite beads can be made by suspension or emulsion polymerizations.

### EXAMPLE 8

### Organic Filler Material

A polymerizable dental material was prepared by stirring at 85°C a liquid mixture of 40 grams of oligomer made following the procedure of Example 1 (e.g., about 20 to about 60, preferably from about 30 to about 50% by wt the organic filler material); 39.25 grams of compound of Example 2 (e.g., about 20 to about 60, preferably from about 30 to about 50% by wt the organic filler material); 20 grams of compound of Example 3 (e.g., about 5 to about 40, preferably from about 10 to about 30% by wt the organic filler material); 0.75 grams of visible light initiating solution (e.g., about 0.005 to about 10, preferably from about 0.05 to about 5% by wt the organic filler material) containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane. This material was subsequently cryogenic ground to form particulate powder containing particles having an average particle size in the range of about 1 to about 150 micrometers. Alternatively, these polymer beads can be made by suspension or emulsion polymerizations.

### EXAMPLE 9

### Composite Filler Material

A polymerizable dental composite material was prepared by mixing a mixture of 51 grams of oligomer made following the procedure of Example 1 (e.g., about 1 to about 25, preferably from about 5 to about 20% by wt the composite filler material); 28 grams of compound of Example 2 (e.g., about 0.5 to about 20, preferably from about 1 to about 10% by wt the composite filler material); 18 grams of compound of Example 3 (e.g., about 0.5 to about 15, preferably from about 1 to about 10% by wt the composite filler material); 59.93 grams of silanated fumed silica (SiO₂) (e.g., about 1 to about 30, preferably from about 5 to about 20% by wt the composite filler material) having an average particles size of from about 0.01 to about 0.04 micrometers; 179.8 grams of silanated barium aluminoflurosilicate glass particles BAFG (e.g., about 20 to about 70, preferably from about 40 to about 60% by wt the composite filler material) having an average particle size of from about 0.1 to about 1 micrometer; 59.93 grams of silanated barium aluminoflurosilicate glass particles BAFG (e.g., about 1 to about 30, preferably from about 5 to about 20% by wt the composite filler material) having an average particle size of from about 1 to about 10 micrometers, 0.08 grams of #115 Phosphor (e.g., about 0.005 to about 5, preferably from about 0.009 to about 0.1% by wt the composite filler material); 0.0192 grams of Lumilux Blue LZ fluorescing agent (dihydroxy terepthalate acid ester) (e.g., about 0.0005 to about 0.1, preferably from about 0.001 to about 0.05 by wt the composite filler material); 0.4 grams of Lucirin-TPO (2,4,6-Trimethylbenzoyldiphenylphosphine oxide) (e.g., about 0.01 to about 5, preferably from about 0.05 to about 1% by wt the composite filler material); and 2.0 grams (0.50%) of visible light initiating solution (e.g., about 0.05 to about 5, preferably from about 0.1 to about 1% by wt the composite filler material) containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane. This composite material was subsequently cryogenic ground to form powders with average particle sizes ranging from about 1 to about 150 micrometers, preferably about 2 to about 50 micrometers. Alternatively, these composite beads can be made by suspension or emulsion polymerizations.

### Printable Polymerizable Compositions

Printable polymerizable compositions are used in a 3D building resin bath of 3D printer to fabricate the dental objects. These compositions may contain acrylate or methacrylate monomers or oligomers, polymers, fillers, pigments, stabilizers and light curable initiators, etc. Preferably, these resins will form flowable liquids at ambient or elevated temperatures and cure rapidly at those temperatures required for different resins to form 3D objects layer-wise. This results in shape-stable three-dimensional objects being formed immediately.

### REFERENCE EXAMPLE 10

### Dental Materials

A polymerizable dental material was prepared by stirring at ambient temperature a liquid mixture of 38 grams of oligomer made following the procedure of Example 1 (e.g., about 15 to about 50, preferably from about 25 to about 40% by wt the dental material); 57 grams of methyl methacrylate (MMA) (e.g., about 30 to about 80, preferably from about 40 to about 70% by wt the dental material); 4 grams of ethylene glycol dimethacrylate (e.g., about 0 to about 15, preferably from about 1 to about 10% by wt the dental material), and 1.0 gram of visible light initiating solution (e.g., about 0.05 to about 10, preferably from about 0.1 to about 5% by wt the dental material) containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane.

### EXAMPLE 10A

### Dental Materials

A polymerizable dental material was prepared by stirring at ambient temperature a liquid mixture of 35.0 grams of oligomer made following the procedure of Example 1 (e.g., about 15 to about 50, preferably from about 20 to about 40% by wt the dental material); 46.0 grams of methyl methacrylate (MMA) (e.g., about 30 to about 60, preferably from about 40 to about 55% by wt the dental material); 10 grams of 2-phenoxyethyl acrylate (e.g., about 0 to about 30, preferably from about 5 to about 20% by wt the dental material); 7.5 grams of a silicone-acrylic-based rubber impact modifier such as S2006 from Mitsubishi Rayon Co. (e.g., about 0.5 to about 20, preferably from about 5 to about 10% by wt the dental material); 1.0 gram of 2,4,6-trimethylbenzoyldiphenylphosphine oxide, (Lucirin TPO available from BASF) (e.g., about 0.005 to about 8, preferably from about 0.05 to about 5% by wt the dental material); and 0.5 gram of visible light initiating solution (e.g., about 0 to about 8, preferably from about 0.05 to about 5% by wt the dental material) containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane.

### EXAMPLE 11

### Dental Materials

A polymerizable dental material was prepared by stirring at ambient temperature a liquid mixture of about 20 to about 30% by wt of oligomer made following the procedure of Example 1; about 60 to about 70% by wt of methyl methacrylate (MMA); about 0.5 to about 10% by wt of ethylene glycol dimethacrylate; about 1 to about 10% by wt of a silicone-acrylic-based rubber impact modifier; about 0.05 to about 5% by wt of visible light initiating solution containing 10-20% (e.g., about 13.3%) camphorquinone (CQ), 15-30% (e.g., about 23.0%) methacrylic acid (MAA), 0.05-5% (e.g., about 1.3%) butylated hydroxytoluene (BHT), 35-55% (e.g., about 46%) N, N-dimethylaminoethylneopentyl acrylate, and 10-20% (e.g., about 16.3%) γ-methacryloxypropyltrimethoxysilane.

### EXAMPLE 12

### Dental Materials

A polymerizable dental material was prepared by stirring at ambient temperature a liquid mixture of about 20 to about 30% by wt of oligomer made following the procedure of Example 5; about 45 to about 55% by wt of methyl methacrylate (MMA); about 5 to about 15% by wt of the polymer D7-99 (manufactured by Dentsply International); about 3 to about 10% by wt of rubber impact modifier S2006 (from Mitsubishi Rayon Co.); about 5 to about 15% by wt of 1,14-tetradecanedimethacrylate: about 0.05 to about 5% by wt of 2,4,6-trimethylbenzoyldiphenylphosphine oxide, (Lucirin TPO available from BASF); and about 0 to about 5% by wt of visible light initiating solution containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane.

### REFERENCE EXAMPLE 13

### Dental Materials

A polymerizable dental material was prepared by stirring at ambient temperature a liquid mixture of about 10 to about 38% by wt of SR368* [Tris(2-Hydroxy Ethyl) Isocyanurate Triacrylate, from Sartomer]; about 40 to about 55% by wt of methyl methacrylate (MMA); about 0 to about 15% by wt of SR399* (Dipentaerythritol pentaacrylate, from Sartomer); about 0 to about 7% by wt of CN121* (Epoxy acrylate oligomer, from Sartomer); about 0 to about 10% by wt of Elvacite 2009 [Poly(methyl methacrylate-co-ethylacrylate), from Sartomer]; about 0 to about 5% by wt of BKY-UV 3530.(Polyether modified acryl functional polydimethyl siloxane); about 0.5 to about 7% by wt of 2,4,6- trimethylbenzoyldiphenylphosphine oxide (Lucirin TPO available from BASF); about 0 to about 5% by wt of visible light initiating solution containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethyineopentyl acrylate, and 5-30% (e.g., about 16.3%) γ-methacryloxypropyltrimethoxysilane.

### EXAMPLE 14

### Dental Materials

A polymerizable dental material was prepared by stirring at ambient temperature a liquid mixture of about 10 to about 30% by wt of SR368* [Tris(2-Hydroxy Ethyl) Isocyanurate Triacrylate, from Sartomer]; about 40 to about 60% by wt of methyl methacrylate (MMA); about 1 to about 10% by wt of SR399* (Dipentaerythritol pentaacrylate, from Sartomer); about 5 to about 15% by wt of polymer D7-99 (manufactured by Dentsply International); about 1 to about 15% by wt of a silicone-acrylic-based rubber impact modifier; about 0.5 to about 10% by wt of 2,4,6- trimethylbenzoyldiphenylphosphine oxide (Lucirin TPO available from BASF); about 0.05 to about 5% by wt of visible light initiating solution containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane.

### REFERENCE EXAMPLE 15

### Dental Materials

A polymerizable dental material was prepared by stirring at ambient temperature a liquid mixture of about 10 to about 30% by wt of monomer CD401 (purchased from Sartomer); about 50 to about 75% by wt of methyl methacrylate (MMA); about 1 to about 10% by wt of SR368* [Tris(2-Hydroxy Ethyl) Isocyanurate Triacrylate, from Sartomer]; about 0.005 to about 5% by wt of 2,4,6- trimethylbenzoyldiphenylphosphine oxide (Lucirin TPO available from BASF); about 0.005 to about 5% by wt of visible light initiating solution containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane.

### REFERENCE EXAMPLE 16

### Dental Materials

A polymerizable dental material was prepared by stirring at ambient temperature a liquid mixture of about 1 to about 10% by wt of monomer CD401 (purchased from Sartomer); about 60 to about 90% by wt of methyl methacrylate (MMA); about 1 to about 10% by wt of SR368* [Tris(2-Hydroxy Ethyl) Isocyanurate Triacrylate, from Sartomer]; about 5 to about 15% by wt of the polymer D7-99 (manufactured by Dentsply International); about 0.005 to about 5% by wt of 2,4,6- trimethylbenzoyldiphenylphosphine oxide (Lucirin TPO available from BASF); about 0.005 to about 5% by wt of visible light initiating solution containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane.

### REFERENCE EXAMPLE 17

### Dental Materials

A polymerizable dental material was prepared by stirring at 85°C a liquid mixture of about 5 to about 18% by wt of oligomer made following the procedure of Example 1; about 25 to about 35% by wt of the compound of Example 2; about 7 to about 18% by wt of the compound of Example 3; about 40 to about 50% by wt of 1,14-tetradecanedimethacrylate, and about 0.005 to about 5% by wt of 2,4,6- trimethylbenzoyldiphenylphosphine oxide (Lucirin TPO available from BASF).

### REFERENCE EXAMPLE 18

### Dental Materials

A polymerizable dental material was prepared by stirring at 85°C a liquid mixture of about 35 to about 50% by wt of oligomer made following the procedure of Example 1; about 45 to about 60% by wt of 7,7,9-trimethyl-4,13-dioxo-3,14 dioxa-5,12-diazahexadecane-1,16-diol dimethacrylate; and about 1 to about 20% by wt of the compound of Example 4; and about 0.05 to about 5% by wt of 2,4,6- trimethylbenzoyldiphenylphosphine oxide (Lucirin TPO available from BASF); about 0 to about 5% by wt of visible light initiating solution containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane.

### REFERENCE EXAMPLE 19

### Dental Materials

A polymerizable dental material was prepared by stirring at 85°C a liquid mixture of about 35 to about 48% by wt of oligomer made following the procedure of Example 1; about 35 to about 48% by wt of 7,7,9-trimethyl-4,13-dioxo-3,14 dioxa-5,12-diazahexadecane-1,16-diol dimethacrylate; about 1 to about 15% by wt of SR368* [Tris(2-Hydroxy Ethyl) Isocyanurate Triacrylate, from Sartomer]; about 5 to about 18% by wt of ethylene glycol dimethacrylate; and about 0.05 to about 5% by wt of 2,4,6- trimethylbenzoyldiphenylphosphine oxide (Lucirin TPO available from BASF); about 0 to about 5% by wt of visible light initiating solution containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane.

### REFERENCE EXAMPLE 20

### Dental Materials

A polymerizable dental material was prepared by stirring at 85°C a liquid mixture of about 20 to about 38% by wt of oligomer made following the procedure of Example 1; about 10 to about 20% by wt of the compound of Example 2; about 1 to about 12% by wt of the compound of Example 3; about 10 to about 28% by wt of 1,14-tetradecanedimethacrylate; about 5 to about 18% by wt of dimethylol tricyclodecane diacrylate; about 5 to about 18% by wt of 7,7,9-trimethyl-4,13-dioxo-3,14 dioxa-5,12-diazahexadecane-1,16-diol dimethacrylate; about 12 to about 28% by wt of Genomer 4256 (aliphatic polyester urethane methacrylate supplied by Rohm America Inc.); and about 0.005 to about 5% by wt of 2,4,6-trimethylbenzoyldiphenylphosphine oxide (Lucirin TPO supplied by BASF).

### REFERENCE EXAMPLE 21

### Dental Materials

A polymerizable dental material was prepared by stirring at 85°C a liquid mixture of about 15 to about 30% by wt of oligomer made following the procedure of Example 1, about 20 to about 35% by wt of the compound of Example 2; about 5 to about 20% by wt of the compound of Example 3; about 1 to about 12% by wt of 7,7,9-trimethyl-4,13-dioxo-5,12-diazahexadecane-1,16-diol dimethacrylate, about 30 to about 45% by wt of 1,14-tetradecanedimethacrylate, and about 0.005 to about 3% by wt of visible light initiating solution containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane.

### REFERENCE EXAMPLE 22

### Dental Materials

A polymerizable dental composite material was prepared by mixing a mixture of about 15 to about 28% by wt of monomer made following the procedure of Example 5; about 10 to about 22% by wt of triethylene glycol dimethacrylate; about 0.5 to about 10% by wt of SR368* [Tris(2-Hydroxy Ethyl) Isocyanurate Triacrylate, from Sartomer]; about 0.005 to about 5% by wt of silanated fumed silica (SiO₂) having an average particles size of from about 0.01 to about 0.04 micrometers; about 55 to about 68% by wt of silanated barium aluminoflurosilicate glass particles BAFG having an average particle size of from about 0.1 to about 1 micrometer; about 0.005 to about 5% by wt of Lumilux Blue LZ fluorescing agent (dihydroxy terepthalate acid ester) and pigments; about 0.005 to about 3% by wt of Lucirin-TPO (2,4,6-Trimethylbenzoyldiphenylphosphine oxide); and about 0.005 to about 3% by wt of visible light initiating solution containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane.

### REFERENCE EXAMPLE 23

### Dental Materials

A polymerizable dental composite material was prepared by mixing a mixture of about 5 to about 18% by wt of monomer made following the procedure of Example 5; about 5 to about 18% by wt of NCO monomer (made by Dentsply Caulk); about 10 to about 22% by wt of triethylene glycol dimethacrylate; about 0.5 to about 10% by wt of SR368* [Tris(2-Hydroxy Ethyl) Isocyanurate Triacrylate, from Sartomer]; about 0.005 to about 3% by wt of silanated fumed silica (SiO₂) having an average particles size of from about 0.01 to about 0.04 micrometers; about 55 to about 65% by wt of composite filler of Example 9; about 0.005 to about 3% by wt of Lumilux Blue LZ fluorescing agent (dihydroxy terepthalate acid ester) and pigments; about 0.005 to about 3% by wt of Lucirin-TPO (2,4,6-Trimethylbenzoyldiphenylphosphine oxide); and about 0.005 to about 3% by wt of visible light initiating solution containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane.

### EXAMPLE 24

### Dental Materials

A polymerizable dental material was prepared by stirring at 85°C a liquid mixture of about 35 to about 48% by wt of oligomer made following the procedure of Example 1; about 35 to about 48% by wt of 7,7,9-trimethyl-4,13-dioxo-3,14 dioxa-5,12-diazahexadecane-1,16-diol dimethacrylate; about 2 to about 18% by wt of methyl methacrylate; about 5 to about 18% by wt of a silicone-acrylic-based rubber impact modifier; and about 0.05 to about 5% by wt of 2,4,6-trimethylbenzoyldiphenylphosphine oxide (Lucirin TPO available from BASF); about 0.005 to about 3% by wt of visible light initiating solution containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane.

### EXAMPLE 25

### Dental Materials

A polymerizable dental material was prepared by stirring at 85°C a liquid mixture of about 15 to about 28% by wt of oligomer made following the procedure of Example 5; about 15 to about 28% by wt of 7,7,9-trimethyl-4,13-dioxo-3,14 dioxa-5,12-diazahexadecane-1,16-diol dimethacrylate; about 30 to about 45% by wt of methyl methacrylate; about 5 to about 15% by wt of a silicone-acrylic-based rubber impact modifier; about 5 to about 18% by wt of the polymer D7-99 (manufactured by Dentsply International); and about 0.005 to about 3% by wt of 2,4,6-trimethylbenzoyldiphenylphosphine oxide (Lucirin TPO available from BASF); about 0.05 to about 3% by wt of visible light initiating solution containing 5-20% (e.g., about 13.3%) camphorquinone (CQ), 10-35% (e.g., about 23.0%) methacrylic acid (MAA), 0.05-5% (e.g., about 1.3%) butylated hydroxytoluene (BHT), 30-60% (e.g., about 46%) N, N-dimethylaminoethylneopentyl acrylate, and 5-30% (e.g., about 16.3%) γ-methacryloxypropyltrimethoxysilane.

### REFERENCE EXAMPLE 26

### Dental Materials

A wax-like polymerizable dental material was prepard by stirring at 75°C a liquid mixture of about 65 to about 88% by wt of bisphenol A proxylate diglycidyl ether, about 20 to about 38% by wt of 1,10 decanediol, 1.0 gram 4-octyloxy-phenyl iodonium hexafluoroantimonate (OPPI), about 0.005 to about 3% by wt of 2,4,6-trimethylbenzoyldiphenylphosphine oxide, (Lucirin TPO made by BASF), about 0.005 to about 3% by wt of pigment concentrates.

### EXAMPLE 27

### Dental Materials

A polymerizable dental material was prepared by stirring at about ambient temperature a liquid mixture of 0 to 50% (e.g., 4 to 45%) of oligomer made following the procedure of Example 1; 40% to 90% (e.g., 50 to 80%) of methyl methacrylate (MMA); 0 to 50% (e.g., 4 to 45%) of various mono to multifunctional (meth)acrylates; 0 to 20% (e.g., 2 to 18%) of PMMA polymer; 0 to 20% (e.g., 2 to 18%) of rubber impact modifiers; 0 to 60% (e.g., 5 to 55%) inorganic or composite fillers; 0 to 10% (e.g., 1 to 9%) pigments and other additives, such as fluorescing agents and inhibitors; and 0.01 to 10% (e.g., 0.1 to 9%) of light initiators.

### EXAMPLE 28

### Dental Materials

A polymerizable dental material was prepared by stirring at about ambient temperature a liquid mixture of 0 to 50% (e.g., 5 to 45%) of oligomer made following the procedure of Example 5; 40 to 90% (e.g., 45 to 85%) of methyl methacrylate (MMA); 0 to 20% (e.g., 2 to 18%) of PMMA polymer; 0 to 20% (e.g., 2 to 18%) of rubber impact modifiers; 0 to 50% (e.g., 5 to 45%) of various mono to multifunctional (meth)acrylates; 0 to 60% (e.g., 5 to 55%) organic, inorganic or composite fillers; 0 to 10% (e.g., 1 to 9%) pigments and other additives, such as fluorescing agents and inhibitors; and 0.01 to 10% (e.g., 0.1 to 8%) of light initiators.

### EXAMPLE 29

### Dental Materials

A polymerizable dental material was prepared by stirring at about ambient temperature a liquid mixture of 0 to 50% (e.g., 5 to 45%) of SR368* [Tris(2-Hydroxy Ethyl) Isocyanurate Triacrylate, from Sartomer]; 40 to 90% (e.g., 50 to 80%) of methyl methacrylate (MMA); 0 to 20% (e.g., 2 to 18%) of PMMA polymer; 0 to 20% (e.g., 2 to 18%) of rubber impact modifiers; 0 to 50% (e.g., 5 to 45%) of various mono to multifunctional (meth)acrylates; 0 to 60% (e.g., 10 to 50%) organic, inorganic or composite fillers; 0 to 10% (e.g., 1 to 9%) pigments and other additives, such as fluorescing agents and inhibitors; and 0.01 to 10% (e.g., 0.1 to 8%) of light initiators.

### REFERENCE EXAMPLE 30

### Dental Materials

A polymerizable dental material was prepared by stirring at about 70°C to about 100°C (e.g., about 85°C) a liquid mixture of 0 to 99.5% (e.g., 10 to 85%, preferably 20 to 75%) of oligomer made following the procedure of Example 1; 0 to 50% (e.g., 5 to 45%) of the compound of Example 2; 0 to 50% (e.g., 5 to 45%) of the compound of Example 3; 0 to 80% (e.g., 20 to 70%) of various mono to multifunctional (meth)acrylates; 0 to 60% (e.g., 10 to 50%) organic, inorganic or composite fillers; 0 to 10% (e.g., 1 to 9%) pigments and other additives, such as fluorescing agents and inhibitors; and 0.01 to 10% (e.g., 0.1 to 8%) of light initiators.

### REFERENCE EXAMPLE 31

### Dental Materials

A polymerizable dental material was prepared by stirring at about 70°C to about 100°C (e.g., about 85°C) a liquid mixture of 0 to 99.5% (e.g., 25 to 75%) of oligomer made following the procedure of Example 1; 0 to 80% (e.g., 20 to 70%) of various mono to multifunctional (meth)acrylates; 0 to 60% (e.g., 10 to 50%) of various inorganic fillers (having an average particle size of from about 0.01 to about 3 micrometer or about 0.1 to about 2.5 micrometer); 0 to 60% (e.g., 10 to 50%) of various composite or organic fillers (having an average particle size of from about 1 to about 100 micrometer or about 5 to about 75 micrometer); 0 to 10% (e.g., 1 to 9%) pigments and other additives, such as fluorescing agents and inhibitors; and 0.01 to 10% (e.g., 0.1 to 9%) of light initiators.

### REFERENCE EXAMPLE 32

### Dental Materials

A polymerizable dental composite material was prepared by mixing a mixture of 0 to 99.5% (e.g., 25 to 75%) of monomer made following the procedure of Example 5; 0 to 80% (e.g., 20 to 70%) of various mono to multifunctional (meth)acrylates; 0 to 60% (e.g., 10 to 45%) of various inorganic fillers (having an average particle size of from about 0.01 to about 3 micrometer or about 0.1 to about 2.5 micrometer); 0 to 60% (e.g., 5 to 45%) of various composite or organic fillers (having an average particle size of from about 1 to about 100 micrometer or about 25 to about 75 micrometer); 0 to 10% (e.g., 1 to 8%) pigments and other additives, such as fluorescing agents and inhibitors; and 0.01 to 10% (e.g., 0.1 to 7%)of light initiators.

### REFERENCE EXAMPLE 33 (PROPHETIC)

### Fabrication of Dental roduct

The material of Example 10 with the addition of pigments is loaded into reservoir of an EnvisionTec printer and sequential voxel planes are projected into the liquid resin in a layer-wise manner as controlled by a computer. This process can be used to form a denture in a layer-by-layer manner. This process produces a denture that can be subsequently added teeth into the formed cavities. Once denture is made, final cured, finished and polished, the denture is delivered to patient.

### EXAMPLE 33A (PROPHETIC)

### Fabrication of Dental Product

The material of Example 10A with the addition of pigments is loaded into reservoir of a SLA based 3D printer and the laser (light) beam traces into the liquid resin in a layer-wise manner as controlled by a computer. This process can be used to form a denture in a layer-by-layer manner. This process produces a denture that can be subsequently added teeth into the formed cavities. If additional layers are needed for teeth, additional reservoirs can be used according to the method mentioned above. Once denture is made, final cured, finished and polished, the denture is delivered to patient.

### REFERENCE EXAMPLE 34 (PROPHETIC)

### Fabrication of Dental Product

The materials of Example 15 and 16 with the addition of pigments are loaded into two separate reservoirs of an envisiontec printer and sequential voxel planes are projected into the first liquid resin (Example 16) in a layer-wise manner as controlled by a computer to form dentin parts of artificial teeth. Formed dentin parts of artificial teeth are removed from this bath. After rinsed with solvent and dried, these dentin parts are immersed into second bath and sequential voxel planes are projected into the second liquid resin (Example 15) in a layer-wise manner as controlled by a computer to form enamel parts on top of dentin parts to form artificial teeth. Finally, artificial teeth are removed from bath, washed and final cured. After polished and finished, these artificial teeth can be used to make denture and other dental devices. This process can be used to mass manufacture artificial teeth and other dental devices.

### REFERENCE EXAMPLE 34A (PROPHETIC)

### Fabrication of Dental Product

The materials of Example 15 and 16 with the addition of pigments are loaded into two separate reservoirs of a SLA based 3D printer and the laser (light) beam traces into the first liquid resin (Example 16) in a layer-wise manner as controlled by a computer to form dentin parts of artificial teeth. Formed dentin parts of artificial teeth are removed from this bath. After rinsed with solvent and dried, these dentin parts are immersed into second bath and sequential voxel planes are projected into the second liquid resin (Example 15) in a layer-wise manner as controlled by a computer to form enamel parts on top of dentin parts to form artificial teeth. Finally, artificial teeth are removed from bath, washed and final cured. After polished and finished, these artificial teeth can be used to make denture and other dental devices. If additional layers are needed for teeth, additional reservoirs can be used according to the method mentioned above. This process can be used to mass manufacture artificial teeth and other dental devices.

### REFERENCEEXAMPLE 35 (PROPHETIC)

### Fabrication of Dental Product

The materials of Example 11, 15 and 16 with the addition of pigments are loaded into three separate reservoirs of an envisiontec printer and sequential voxel planes are projected into the first liquid resin (Example 11) in a layer-wise manner as controlled by a computer to form denture bases. Formed denture bases are removed from this bath. After rinsed with solvent and dried, these denture bases are immersed into second bath and sequential voxel planes are projected into the second liquid resin (Example 16) in a layer-wise manner as controlled by a computer to form dentin parts of artificial teeth on top of denture bases. Formed denture bases with dentin parts are removed from this bath. After rinsed with solvent and dried, these parts are immersed into third bath and sequential voxel planes were projected into the second liquid resin (Example 15) in a layer-wise manner as controlled by a computer to form enamel parts on top of dentin parts to form dentures. If additional layers are needed for denture bases or teeth, additional reservoirs can be used according to the method mentioned above. Finally, dentures are removed from bath, washed and final cured. Once dentures are made, final cured, finished and polished, the dentures are delivered to patients.

### EXAMPLE 35A (PROPHETIC)

### Fabrication of Dental Product

The materials of Example 10A, 15 and 16 with the addition of pigments are loaded into three separate reservoirs of a SLA based 3D printer and the laser (light) beams traces into the first liquid resin (Example 10A) in a layer-wise manner as controlled by a computer to form denture bases. Formed denture bases are removed from this bath. After rinsed with solvent and dried, these denture bases are immersed into second bath and subsequently laser (light) beam traces into the second liquid resin (Example 16) in a layer-wise manner as controlled by a computer to form dentin parts of artificial teeth on top of denture bases. Formed denture bases with dentin parts are removed from this bath. After rinsed with solvent and dried, these parts are immersed into third bath and the laser (light) beams traces into the second liquid resin (Example 15) in a layer-wise manner as controlled by a computer to form enamel parts on top of dentin parts to form dentures. Following the same approach, additional layers can be built if desired. Finally, dentures are removed from bath, washed and final cured. Once dentures are made, final cured, finished and polished, the dentures are delivered to patients.

### REFERENCE EXAMPLE 36 (PROPHETIC)

### Fabrication of Dental Product

The materials of Example 18, and 19 (two shades) with the addition of pigments are loaded into three separate heated reservoirs of an EnvisionTec printer and sequential voxel planes are projected into the first liquid resin (Example 18) in a layer-wise manner as controlled by a computer to form denture bases. Formed denture bases are removed from this bath. After rinsed with solvent and dried, these denture bases are immersed into second bath (dentin shade of example 19) and sequential voxel planes are projected into the second liquid resin (dentin shade of example 19) in a layer-wise manner as controlled by a computer to form dentin parts of artificial teeth on top of denture bases. Formed denture bases with dentin parts are removed from this bath. After rinsed with solvent and dried, these parts are immersed into third bath and sequential voxel planes are projected into the second liquid resin (enamel shade of example 19) in a layer-wise manner as controlled by a computer to form enamel parts on top of dentin parts to form dentures. Finally, dentures are removed from bath, washed and final cured. Once dentures are made, final cured, finished and polished, the dentures are delivered to patients.

### REFERENCE EXAMPLE 36A (PROPHETIC)

### Fabrication of Dental Product

The materials of Example 18, and 19 (two shades) with the addition of pigments are loaded into three separate heated reservoirs of a SLA based 3D printer and the laser (light) beams traces into the first liquid resin (Example 18) in a layer-wise manner as controlled by a computer to form denture bases. Formed denture bases are removed from this bath. After rinsed with solvent and dried, these denture bases are immersed into second bath (dentin shade of example 19) and sequential voxel planes and the laser (light) beams traces into the second liquid resin (dentin shade of example 19) in a layer-wise manner as controlled by a computer to form dentin parts of artificial teeth on top of denture bases. Formed denture bases with dentin parts are removed from this bath. After rinsed with solvent and dried, these parts are immersed into third bath and the laser (light) beams traces into the second liquid resin (enamel shade of example 19) in a layer-wise manner as controlled by a computer to form enamel parts on top of dentin parts to form dentures. Finally, dentures are removed from bath, washed and final cured. Once dentures are made, final cured, finished and polished, the dentures are delivered to patients.

### REFERENCE EXAMPLE 37 (PROPHETIC)

### Fabrication of Dental Product

The materials of Example 22 (enamel and dentin shaded) are loaded into two separate reservoirs (heated as needed) of an EnvisionTec printer and sequential voxel planes are projected into the first liquid resin (dentin shaded) in a layer-wise manner as controlled by a computer to form crown shapes. Formed crown parts are removed from this bath. After rinsed with solvent and dried, these crown parts are immersed into second bath and sequential voxel planes are projected into the second liquid resin (enamel shaded) in a layer-wise manner as controlled by a computer to form enamel parts on top of dentin parts to form final crowns. Finally, crowns are removed from bath, washed and final cured. This process can be used to mass manufacture crowns, bridges, artificial teeth and other dental devices.

Optional, sealer may be applied to these crowns, and then cured in a light-curing unit for 1 to 10 minutes. This curing step produces final crown products, which can be relined or cemented on a crown-prepped tooth in a patient's mouth.

### REFERENCE EXAMPLE 37A (PROPHETIC)

### Fabrication of Dental Product

The materials of Example 22 (enamel and dentin shaded) are loaded into two separate reservoirs (heated as needed) of a SLA based 3D printer and the laser (light) beams traces into the first liquid resin (dentin shaded) in a layer-wise manner as controlled by a computer to form crown shapes. Formed crown parts are removed from this bath. After rinsed with solvent and dried, these crown parts are immersed into second bath and the laser (light) beams traces into the second liquid resin (enamel shaded) in a layer-wise manner as controlled by a computer to form enamel parts on top of dentin parts to form final crowns. Finally, crowns are removed from bath, washed and final cured. This process can be used to mass manufacture crowns, bridges, artificial teeth and other dental devices.

Optional, sealer may be applied to these crowns, and then cured in a light-curing unit for 1 to 10 minutes. This curing step produces final crown products, which can be relined or cemented on a crown-prepped tooth in a patient's mouth.

### REFERENCE EXAMPLE 38 (PROPHETIC)

### Fabrication of Dental Product

The materials of Example 20 (with the addition of pigments and red fibers), and 23 (two shades) are loaded into three separate heated reservoirs of an EnvisionTec printer and sequential voxel planes are projected into the first liquid resin (Example 20) in a layer-wise manner as controlled by a computer to form denture bases. Formed denture bases are removed from this bath. After rinsed with solvent and dried, these denture bases are immersed into second bath (dentin shade of example 23) and sequential voxel planes are projected into the second liquid resin (dentin shade of example 23) in a layer-wise manner as controlled by a computer to form dentin parts of artificial teeth on top of denture bases. Formed denture bases with dentin parts are removed from this bath. After rinsed with solvent and dried, these parts are immersed into third bath and sequential voxel planes are projected into the second liquid resin (enamel shade of example 23) in a layer-wise manner as controlled by a computer to form enamel parts on top of dentin parts to form dentures. Finally, dentures are removed from bath, washed and final cured. Once dentures are made, final cured, finished and polished, the dentures are delivered to patients.

### REFERENCE EXAMPLE 38A (PROPHETIC)

### Fabrication of Dental Product

The materials of Example 20 (with the addition of pigments and red fibers), and 23 (two shades) are loaded into three separate heated reservoirs of a SLA based 3D printer and the laser (light) beams traces into the first liquid resin (Example 20) in a layer-wise manner as controlled by a computer to form denture bases. Formed denture bases are removed from this bath. After rinsed with solvent and dried, these denture bases are immersed into second bath (dentin shade of example 23) and the laser (light) beams traces into the second liquid resin (dentin shade of example 23) in a layer-wise manner as controlled by a computer to form dentin parts of artificial teeth on top of denture bases. Formed denture bases with dentin parts are removed from this bath. After rinsed with solvent and dried, these parts are immersed into third bath and the laser (light) beams traces into the second liquid resin (enamel shade of example 23) in a layer-wise manner as controlled by a computer to form enamel parts on top of dentin parts to form dentures. Finally, dentures are removed from bath, washed and final cured. Once dentures are made, final cured, finished and polished, the dentures are delivered to patients.

### REFERENCE EXAMPE 39 (PROPHETIC)

### Fabrication of Dental Models and Appliances

Materials such as light (irradiation) polymerizable epoxies (such as material in example 26) and silicones may be loaded into separate, and optionally heated reservoirs of an EnvisionTec Printer and sequential voxel planes are projected into the first liquid bath in a layer-wise manner as controlled by a computer to form models, appliances or accessory products in the fabrication of restorations or appliances. Formed models, appliances are removed from this bath. After optional rinsing with solvent and drying, these models, appliances are immersed into subsequent baths for the addition of other layers (for example differently colored) and sequential voxel planes are projected into these liquid resin baths in a layer-wise manner as controlled by a computer to form layered appliances. Formed models and appliances are removed from these subsequent baths. Finally, appliances may be post processed after cleaning, for delivery to a dental professional or patient.

It will be further appreciated that functions or structures of a plurality of components or steps may be combined into a single component or step, or the functions or structures of one-step or component may be split among plural steps or components. The present invention contemplates all of these combinations. Unless stated otherwise, dimensions and geometries of the various structures depicted herein are not intended to be restrictive of the invention, and other dimensions or geometries are possible. In addition, while a feature of the present invention may have been described in the context of only one of the illustrated embodiments, such feature may be combined with one or more other features of other embodiments, for any given application. It will also be appreciated from the above that the fabrication of the unique structures herein and the operation thereof also constitute methods in accordance with the present invention. The present invention also encompasses intermediate and end products resulting from the practice of the methods herein. The use of "comprising" or "including" also contemplates embodiments that "consist essentially of" or "consist of" the recited feature.

The explanations and illustrations presented herein are intended to acquaint others skilled in the art with the invention, its principles, and its practical application. Those skilled in the art may adapt and apply the invention in its numerous forms, as may be best suited to the requirements of a particular use. Accordingly, the specific embodiments of the present invention as set forth are not intended as being exhaustive or limiting of the invention. The scope of the invention should, therefore, be determined not with reference to the above description, but should instead be determined with reference to the appended claims, along with the full scope of equivalens to which such claims are entitled.

## Claims

1. A method for making a three-dimensional dental prosthesis using a printable composition comprising:
a mixture of
at least 1 % of methyl methacrylate and/or ethyl methacrylate;
5 to 10% a silicone-acrylic-based rubber impact modifier wherein the core is silicone and the shell is PMMA acrylic, which does not dissolve but swells and forms a colloid at room temperature or elevated temperature;
1 to 90% of at least one mono or multifunctional (meth)acrylate;
0 to 60% of at least one inorganic filler;
0 to 60% of at least one organic fillers;
0 to 10% pigments, and
0.01 to 10% of light initiators,
said method either
(A) comprising the steps of
a. loading the polymerizable liquid resin material or heated resin material as a liquid into a resin bath of a 3D printer;
b. applying sequential voxel planes into the liquid resin or heated resin to form a first layer of material, which polymerizes into a solid;
c. applying one or more successive layers of the polymerized material until a predetermined shape is formed,
or
(B) comprising the steps of
a. loading the polymerizable liquid resin material or heated resin material as a liquid into a resin bath of a 3D printer based on stereolithography or other light irradiations;
b. using laser beam or light irradiation tracing out the shape of each layer of the liquid resin or heated resin to form a polymerized solid;
c. applying one or more successive layers of the polymerized material until a predetermined shape is formed.

2. The method of claim 1, wherein the at least one inorganic filler has an average particle size of from 0.01 to 3 micrometer.

3. The method of claim 1, wherein the at least one organic filler has an average particle size of from 1 to 100 micrometer.

4. The method of claim 1, wherein the 3D printer is a stereolithography 3D printer of a digital light processing 3D printer.

5. The method for making a three-dimensional dental prosthesis according to claim 1 to 4, wherein the method (A) further comprising the steps of
d. washing and/or transferring the formed shape into a separate resin bath, which has different shade or/and different physical properties, to build additional layer of materials on the surface of formed shape layer by layer according to step a) to c),
e. optionally, repeat step d) as needed.

6. The method for making a three-dimensional dental prosthesis according to claim 1 to 4, wherein the method (B) further comprising the steps of
d. washing and/or transferring the formed shape into a separate resin bath, which has different shade or/and different physical properties, to build additional layer of materials on the surface of formed shape layer by layer according to step a) to c),
e. optionally, repeat step d) as needed.

7. A method for making a three-dimensional dental prosthesis according to claim 1 comprising:
a mixture of
10 to 80% of methyl methacrylate and ethyl methacrylate;
5 to 10% a silicone-acrylic-based rubber impact modifier;
1 to 20% of at least one multifunctional (meth)acrylate;
0 to 60% of at least one inorganic filler;
0 to 60% of at least one organic fillers.

8. A method for making a three-dimensional dental prosthesis using the composition defined in claim 1 comprising the steps of
a. loading a first polymerizable liquid resin material or heated resin material as a liquid into a resin bath of a 3D printer;
b. loading a second polymerizable liquid resin material or heated resin material as a liquid into a second resin bath of the 3D printer, the second polymerizable liquid resin material or heated resin material being different than the first polymerizable liquid resin material or heated resin material;
c. using laser beam or light irradiation tracing out the shape of at least one layer of the first polymerizable liquid resin material or heated resin material to form at least a first portion of a first polymerized solid;
d. using laser beam or light irradiation tracing out the shape of at least one layer of the second polymerizable liquid resin material or heated resin material to form at least a second portion of the polymerized solid
e. washing the first portion of the first polymerized solid and/or the second portion of the second polymerized solid in a solvent;
f. forming a predetermined shape from the at least one layer of the first portion of the polymerized solid and the at least one layer of the second portion of the second polymerized solid.

9. A method for making a three-dimensional dental prosthesis using the composition defined in claim 1 comprising the steps of
a. loading a first polymerizable liquid resin material or heated resin material as a liquid into a resin bath of a 3D printer;
b. using a laser beam or light irradiation tracing out the shape of a first layer of the first polymerizable liquid resin material or heated resin material to form at least a first portion of a first polymerized solid;
c. applying one or more successive layers of the first polymerizable liquid resin material or heated resin material until a first predetermined polymerized shape is formed;
d. loading a second polymerizable liquid resin material or heated resin material as a liquid into a second resin bath of the 3D printer, the second polymerizable liquid resin material or heated resin material being different than the first polymerizable liquid resin material or heated resin material;
e. immersing the formed first predetermined polymerized shape in the second polymerizable liquid resin material or heated resin material in the second resin bath;
f. using a laser beam or light irradiation, tracing out the shape of a first layer of the second polymerizable liquid resin material or heated resin material to form at least a first portion of a second polymerized solid on the formed first predetermined polymerized shape;
g. applying one or more successive layers of the first polymerizable liquid resin material or heated resin material until a resultant predetermined polymerized shape is formed.

10. The method of claim 9, further comprising the step of rinsing the formed first predetermined polymerized shape with a solvent prior to being immersed in the second polymerizable liquid resin material or heated resin material; or further comprising the step of rinsing the resultant predetermined polymerized shape with a solvent; or wherein the steps of using the laser beam or light irradiation, the formed first predetermined polymerized shape, the formed second predetermined polymerized shape, or both are partially cured, preferably further comprising the step of fully curing the partially cured resultant predetermined polymerized shape; or wherein:
(i) the method further comprises the step of rinsing the first predetermined polymerized shape with a solvent prior to being immersed in the second polymerizable liquid resin material or heated resin material;
(ii) the method further comprises the step of rinsing the rinsing the formed resultant predetermined polymerized shape with a solvent;
(iii) wherein the steps of using the laser beam or light irradiation, the formed first predetermined polymerized shape, the formed second predetermined polymerized shape, or both are partially cured;
(iv) the method further comprises the step of fully curing the partially cured resultant predetermined polymerized shape.

## Patentansprüche

1. Verfahren zum Herstellen einer dreidimensionalen Zahnprothese unter Verwendung einer druckbaren Zusammensetzung, umfassend:
eine Mischung aus
mindestens 1 % Methylmethacrylat und/oder Ethylmethacrylat;
5 bis 10 % Schlagzähmodifizierungsmittel aus Kautschuk auf Silikon-Acryl-Basis, wobei der Kern Silikon ist und die Hülle PMMA-Acryl ist, der sich nicht löst, sondern quillt und bei Raumtemperatur oder erhöhter Temperatur ein Kolloid bildet;
1 bis 90 % mindestens eines mono- oder multifunktionellen (Meth)acrylats;
0 bis 60 % mindestens eines anorganischen Füllstoffs;
0 bis 60 % mindestens eines organischen Füllstoffs;
0 bis 10 % Pigmente, und
0,01 bis 10 % Lichtinitiatoren,
wobei das Verfahren entweder umfasst:
(A) die Schritte:
a. Laden des polymerisierbaren flüssigen Harzmaterials oder erhitzten Harzmaterials als Flüssigkeit in ein Harzbad eines 3D-Druckers;
b. Aufbringen aufeinanderfolgender Voxelebenen in das flüssige Harz oder erhitzte Harz, um eine erste Materialschicht zu bilden, die zu einem Feststoff polymerisiert;
c. Aufbringen einer oder mehrerer aufeinanderfolgender Schichten des polymerisierten Materials, bis eine vorbestimmte Form gebildet ist,
oder
(B) die Schritte:
a. Laden des polymerisierbaren flüssigen Harzmaterials oder erhitzten Harzmaterials als Flüssigkeit in ein Harzbad eines 3D-Druckers auf Basis von Stereolithographie oder anderen Lichtbestrahlungen;
b. Verwenden von Laserstrahl- oder Lichtbestrahlung, die die Form jeder Schicht des flüssigen Harzes oder erhitzten Harzes verfolgt, um einen polymerisierten Feststoff zu bilden;
c. Aufbringen einer oder mehrerer aufeinanderfolgender Schichten des polymerisierten Materials, bis eine vorbestimmte Form gebildet ist.

2. Verfahren nach Anspruch 1, wobei der mindestens eine anorganische Füllstoff eine durchschnittliche Teilchengröße von 0,01 bis 3 Mikrometer aufweist.

3. Verfahren nach Anspruch 1, wobei der mindestens eine organische Füllstoff eine durchschnittliche Teilchengröße von 1 bis 100 Mikrometer aufweist.

4. Verfahren nach Anspruch 1, wobei der 3D-Drucker ein Stereolithographie-3D-Drucker eines 3D-Druckers mit digitaler Lichtverarbeitung ist.

5. Verfahren zum Herstellen einer dreidimensionalen Zahnprothese nach Anspruch 1 bis 4, wobei das Verfahren (A) ferner die Schritte umfasst:
d. Waschen und/oder Überführen der geformten Form in ein separates Harzbad, das unterschiedliche Farbnuancen oder/und unterschiedliche physikalische Eigenschaften aufweist, um eine zusätzliche Schicht von Materialien auf der Oberfläche der geformten Form Schicht für Schicht gemäß Schritt a) bis c) aufzubauen,
e. optional bedarfsgesteuertes Wiederholen von Schritt d).

6. Verfahren zum Herstellen einer dreidimensionalen Zahnprothese nach Anspruch 1 bis 4, wobei das Verfahren (B) ferner die Schritte umfasst:
d. Waschen und/oder Überführen der geformten Form in ein separates Harzbad, das unterschiedliche Farbnuancen oder/und unterschiedliche physikalische Eigenschaften aufweist, um eine zusätzliche Schicht von Materialien auf der Oberfläche der geformten Form Schicht für Schicht gemäß Schritt a) bis c) aufzubauen,
e. optional bedarfsgesteuertes Wiederholen von Schritt d).

7. Verfahren zum Herstellen einer dreidimensionalen Zahnprothese nach Anspruch 1, umfassend:
eine Mischung aus
10 bis 80 % Methylmethacrylat und Ethylmethacrylat;
5 bis 10 % Schlagzähmodifizierungsmittel aus Kautschuk auf Silikon-Acryl-Basis;
1 bis 20 % mindestens eines multifunktionellen (Meth)acrylats;
0 bis 60 % mindestens eines anorganischen Füllstoffs;
0 bis 60 % mindestens eines organischen Füllstoffes.

8. Verfahren zum Herstellen einer dreidimensionalen Zahnprothese unter Verwendung der Zusammensetzung nach Anspruch 1, umfassend die Schritte:
a. Laden eines ersten polymerisierbaren flüssigen Harzmaterials oder erhitzten Harzmaterials als Flüssigkeit in ein Harzbad eines 3D-Druckers;
b. Laden eines zweiten polymerisierbaren flüssigen Harzmaterials oder erhitzten Harzmaterials als Flüssigkeit in ein zweites Harzbad des 3D-Druckers, wobei das zweite polymerisierbare flüssige Harzmaterial oder erhitzte Harzmaterial sich von dem ersten polymerisierbaren flüssigen Harzmaterial oder erhitzten Harzmaterial unterscheidet;
c. Verwenden von Laserstrahl- oder Lichtbestrahlung, die die Form mindestens einer Schicht des ersten polymerisierbaren flüssigen Harzmaterials oder erhitzten Harzmaterials verfolgt, um mindestens einen ersten Teil eines ersten polymerisierten Feststoffs zu bilden;
d. Verwenden von Laserstrahl- oder Lichtbestrahlung, die die Form mindestens einer Schicht des zweiten polymerisierbaren flüssigen Harzmaterials oder erhitzten Harzmaterials verfolgt, um mindestens einen zweiten Teil des polymerisierten Feststoffs zu bilden
e. Waschen des ersten Teils des ersten polymerisierten Feststoffs und/oder des zweiten Teils des zweiten polymerisierten Feststoffs in einem Lösungsmittel;
f. Bilden einer vorbestimmten Form aus der mindestens einen Schicht des ersten Teils des polymerisierten Feststoffs und der mindestens einen Schicht des zweiten Teils des zweiten polymerisierten Feststoffs.

9. Verfahren zum Herstellen einer dreidimensionalen Zahnprothese unter Verwendung der Zusammensetzung nach Anspruch 1, umfassend die Schritte:
a. Laden eines ersten polymerisierbaren flüssigen Harzmaterials oder erhitzten Harzmaterials als Flüssigkeit in ein Harzbad eines 3D-Druckers;
b. Verwenden von Laserstrahl- oder Lichtbestrahlung, die die Form einer ersten Schicht des ersten polymerisierbaren flüssigen Harzmaterials oder erhitzten Harzmaterials verfolgt, um mindestens einen ersten Teil eines ersten polymerisierten Feststoffs zu bilden;
c. Aufbringen einer oder mehrerer aufeinanderfolgender Schichten des ersten polymerisierbaren flüssigen Harzmaterials oder erhitzten Harzmaterials, bis eine erste vorbestimmte polymerisierte Form gebildet ist;
d. Laden eines zweiten polymerisierbaren flüssigen Harzmaterials oder erhitzten Harzmaterials als Flüssigkeit in ein zweites Harzbad des 3D-Druckers, wobei das zweite polymerisierbare flüssige Harzmaterial oder erhitzte Harzmaterial sich von dem ersten polymerisierbaren flüssigen Harzmaterial oder erhitzten Harzmaterial unterscheidet;
e. Eintauchen der gebildeten ersten vorbestimmten polymerisierten Form in das zweite polymerisierbare flüssige Harzmaterial oder erhitzte Harzmaterial in dem zweiten Harzbad;
f. Verwenden von Laserstrahl- oder Lichtbestrahlung, die die Form einer ersten Schicht des zweiten polymerisierbaren flüssigen Harzmaterials oder erhitzten Harzmaterials verfolgt, um mindestens einen ersten Teil eines zweiten polymerisierten Feststoffs auf der gebildeten ersten vorbestimmten polymerisierten Form zu bilden;
g. Aufbringen einer oder mehrerer aufeinanderfolgender Schichten des ersten polymerisierbaren flüssigen Harzmaterials oder erhitzten Harzmaterials, bis eine resultierende vorbestimmte polymerisierte Form gebildet ist.

10. Verfahren nach Anspruch 9, ferner umfassend den Schritt des Spülens der gebildeten ersten vorbestimmten polymerisierten Form mit einem Lösungsmittel vor dem Eintauchen in das zweite polymerisierbare flüssige Harzmaterial oder erhitzte Harzmaterial; oder ferner umfassend den Schritt des Spülens der resultierenden vorbestimmten polymerisierten Form mit einem Lösungsmittel; oder wobei die Schritte des Verwendens der Laserstrahl- oder Lichtbestrahlung die gebildete erste vorbestimmte polymerisierte Form, die gebildete zweite vorbestimmte polymerisierte Form oder beide teilweise gehärtet werden, vorzugsweise ferner umfassend den Schritt des vollständigen Härtens der teilweise gehärteten resultierenden vorbestimmten polymerisierten Form; oder wobei:
(i) das Verfahren ferner den Schritt des Spülens der ersten vorbestimmten polymerisierten Form mit einem Lösungsmittel vor dem Eintauchen in das zweite polymerisierbare flüssige Harzmaterial oder erhitzte Harzmaterial umfasst;
(ii) das Verfahren ferner den Schritt des Spülens des Spülens der gebildeten resultierenden vorbestimmten polymerisierten Form mit einem Lösungsmittel umfasst;
(iii) wobei die Schritte des Verwendens der Laserstrahl- oder der Lichtbestrahlung, die geformte erste vorbestimmte polymerisierte Form, die geformte zweite vorbestimmte polymerisierte Form oder beide teilweise gehärtet werden;
(iv) das Verfahren ferner den Schritt des vollständigen Aushärtens der teilweise ausgehärteten resultierenden vorbestimmten polymerisierten Form umfasst.

## Revendications

1. Procédé de fabrication d'une prothèse dentaire tridimensionnelle à l'aide d'une composition imprimable comprenant :
un mélange de
au moins 1 % de méthacrylate de méthyle et/ ou de méthacrylate d'éthyle ;
5 à 10 % d'un modificateur d'impact de caoutchouc à base de silicone-acrylique dans lequel le noyau est du silicone et l'enveloppe est de l'acrylique PMMA, qui ne se dissout pas mais gonfle et forme un colloïde à température ambiante ou à température élevée ;
1 à 90 % d'au moins un (méth)acrylate mono ou multifonctionnel ;
0 à 60 % d'au moins une charge inorganique ;
0 à 60 % d'au moins une charge organique ;
de 0 à 10 % de pigments, et
de 0,01 à 10 % d'initiateurs de lumière,
ledit procédé soit
(A) comprenant les étapes de
a. le chargement du matériau de résine liquide polymérisable ou du matériau de résine chauffée en tant que liquide dans un bain de résine d'une imprimante 3D ;
b. l'application de plans de voxels séquentiels dans la résine liquide ou la résine chauffée pour former une première couche de matériau, qui se polymérise en un solide ;
c. l'application d'une ou plusieurs couches successives du matériau polymérisé jusqu'à ce qu'une forme prédéterminée soit formée,
ou
(B) comprenant les étapes de
a. le chargement du matériau de résine liquide polymérisable ou du matériau de résine chauffée en tant que liquide dans un bain de résine d'une imprimante 3D sur base d'une stéréolithographie ou d'autres irradiations de lumière ;
b. l'utilisation d'un faisceau laser ou d'une irradiation de lumière traçant la forme de chaque couche de la résine liquide ou de la résine chauffée pour former un solide polymérisé ;
c. l'application d'une ou plusieurs couches successives du matériau polymérisé jusqu'à ce qu'une forme prédéterminée soit formée.

2. Procédé selon la revendication 1, dans lequel l'au moins une charge inorganique a une taille moyenne de particules de 0,01 à 3 micromètres.

3. Procédé selon la revendication 1, dans lequel l'au moins une charge organique a une taille de particule moyenne de 1 à 100 micromètres.

4. Procédé selon la revendication 1, dans lequel l'imprimante 3D est une imprimante 3D à stéréolithographie d'une imprimante 3D de traitement de lumière numérique.

5. Procédé de fabrication d'une prothèse dentaire tridimensionnelle selon les revendications 1 à 4, dans lequel le procédé (A) comprend en outre les étapes de
d. le lavage et/ ou le transfert de la forme façonnée dans un bain de résine séparé, qui a des nuances différentes et/ ou des propriétés physiques différentes, pour construire une couche supplémentaire de matériaux sur la surface de la forme façonnée couche par couche selon les étapes a) à c),
e. répéter éventuellement l'étape d) au besoin.

6. Procédé pour la fabrication d'une prothèse dentaire tridimensionnelle selon les revendications 1 à 4, dans lequel le procédé (B) comprend en outre les étapes de
d. le lavage et/ ou le transfert de la forme façonnée dans un bain de résine séparé, qui a des nuances différentes et/ ou des propriétés physiques différentes, pour construire une couche supplémentaire de matériaux sur la surface de la forme façonnée couche par couche selon les étapes a) à c),
e. répéter éventuellement l'étape d) au besoin.

7. Procédé pour la fabrication d'une prothèse dentaire tridimensionnelle selon la revendication 1 comprenant :
un mélange de
10 à 80 % de méthacrylate de méthyle et de méthacrylate d'éthyle ;
5 à 10 % d'un modificateur d'impact en caoutchouc à base de silicone acrylique ;
1 à 20 % d'au moins un (méth)acrylate multifonctionnel ;
0 à 60 % d'au moins une charge inorganique ;
0 à 60 % d'au moins une charge organique.

8. Procédé de fabrication d'une prothèse dentaire tridimensionnelle à l'aide de la composition définie dans la revendication 1, comprenant les étapes de
a. le chargement d'un premier matériau de résine liquide polymérisable ou d'un matériau de résine chauffée en tant que liquide dans un bain de résine d'une imprimante 3D ;
b. le chargement d'un deuxième matériau de résine liquide polymérisable ou d'un matériau de résine chauffée en tant que liquide dans un deuxième bain de résine de l'imprimante 3D, le deuxième matériau de résine liquide polymérisable ou matériau de résine chauffée étant différent du premier matériau de résine liquide polymérisable ou du matériau de résine chauffée ;
c. l'utilisation d'un faisceau laser ou d'une irradiation de lumière traçant la forme d'au moins une couche du premier matériau de résine liquide polymérisable ou du matériau de résine chauffée pour former au moins une première partie d'un premier solide polymérisé ;
d. l'utilisation d'un faisceau laser ou d'une irradiation de lumière traçant la forme d'au moins une couche du deuxième matériau de résine liquide polymérisable ou du matériau de résine chauffée pour former au moins une deuxième partie du solide polymérisé
e. le lavage de la première partie du premier solide polymérisé et/ ou de la deuxième partie du deuxième solide polymérisé dans un solvant ;
f. la formation d'une forme prédéterminée à partir de l'au moins une couche de la première partie du solide polymérisé et de l'au moins une couche de la deuxième partie du deuxième solide polymérisé.

9. Procédé de fabrication d'une prothèse dentaire tridimensionnelle à l'aide de la composition définie dans la revendication 1, comprenant les étapes de
a. le chargement d'un premier matériau de résine liquide polymérisable ou d'un matériau de résine chauffée en tant que liquide dans un bain de résine d'une imprimante 3D ;
b. l'utilisation d'un faisceau laser ou d'irradiation de lumière traçant la forme d'une première couche du premier matériau de résine liquide polymérisable ou du matériau de résine chauffée pour former au moins une première partie d'un premier solide polymérisé ;
c. l'application d'une ou plusieurs couches successives du premier matériau de résine liquide polymérisable ou du matériau de résine chauffée jusqu'à ce qu'une première forme polymérisée prédéterminée soit formée ;
d. le chargement d'un deuxième matériau de résine liquide polymérisable ou d'un matériau de résine chauffée en tant que liquide dans un deuxième bain de résine de l'imprimante 3D, le deuxième matériau de résine liquide polymérisable ou matériau de résine chauffée étant différent du premier matériau de résine liquide polymérisable ou du matériau de résine chauffée ;
e. l'immersion de la première forme polymérisée prédéterminée formée dans le deuxième matériau de résine liquide polymérisable ou du matériau de résine chauffée dans le deuxième bain de résine ;
f. l'utilisation d'un faisceau laser ou d'une irradiation de lumière, traçant la forme d'une première couche du deuxième matériau de résine liquide polymérisable ou du matériau de résine chauffée pour former au moins une première partie d'un deuxième solide polymérisé sur la première forme polymérisée prédéterminée formée ;
g. l'application d'une ou plusieurs couches successives du premier matériau de résine liquide polymérisable ou du matériau de résine chauffée jusqu'à ce qu'une forme polymérisée prédéterminée résultante soit formée.

10. Procédé selon la revendication 9, comprenant en outre l'étape de rinçage de la première forme polymérisée prédéterminée formée avec un solvant avant d'être immergée dans le deuxième matériau de résine liquide polymérisable ou du matériau de résine chauffée ; ou comprenant en outre l'étape de rinçage de la forme polymérisée prédéterminée résultante avec un solvant ; ou dans lequel les étapes d'utilisation du faisceau laser ou de l'irradiation de lumière, la première forme polymérisée prédéterminée formée, la deuxième forme polymérisée prédéterminée formée, ou les deux sont partiellement durcies, comprenant en outre de préférence l'étape de durcissement complet de la forme polymérisée prédéterminée partiellement durcie résultante ; ou dans lequel :
(i) le procédé comprend en outre l'étape de rinçage de la première forme polymérisée prédéterminée avec un solvant avant d'être immergée dans le deuxième matériau de résine liquide polymérisable ou le matériau de résine chauffée ;
(ii) le procédé comprend en outre l'étape de rinçage du rinçage de la forme polymérisée prédéterminée résultante formée avec un solvant ;
(iii) dans lequel les étapes d'utilisation du faisceau laser ou de l'irradiation de lumière, la première forme polymérisée prédéterminée formée, la deuxième forme polymérisée prédéterminée formée, ou les deux sont partiellement durcies ;
(iv) le procédé comprend en outre l'étape de durcissement complet de la forme polymérisée prédéterminée résultante partiellement durcie.
